(19) Europäisches Patentamt
European Patent Office
Office européen des brevets

(11) Veröffentlichungsnummer: **0 156 255**
**A2**

# (12) EUROPÄISCHE PATENTANMELDUNG

(21) Anmeldenummer: **85102929.8**

(22) Anmeldetag: **14.03.85**

(51) Int. Cl.⁴: **C 07 D 405/12**
A 01 N 47/38

(30) Priorität: **24.03.84 DE 3410925**

(43) Veröffentlichungstag der Anmeldung:
**02.10.85 Patentblatt 85/40**

(84) Benannte Vertragsstaaten:
**AT BE CH DE FR GB IT LI NL SE**

(71) Anmelder: **BASF Aktiengesellschaft**
**Carl-Bosch-Strasse 38**
**D-6700 Ludwigshafen(DE)**

(72) Erfinder: **Rentzea, Costin, Dr. Dipl.-Ing.**
**Richard-Kuhn-Strasse 1-3**
**D-6900 Heidelberg(DE)**

(72) Erfinder: **Karbach, Stefan, Dr.**
**Sperlingsgasse 3**
**D-6700 Ludwigshafen(DE)**

(72) Erfinder: **Ammermann, Eberhard, Dr.**
**Sachsenstrasse 3**
**D-6700 Ludwigshafen(DE)**

(72) Erfinder: **Pommer, Ernst-Heinrich, Dr.**
**Berliner Platz 7**
**D-6703 Limburgerhof(DE)**

(54) **N-((2,3-Dihydrobenzofuran)-2yl)-azolylharnstoffe und diese enthaltende Fungizide.**

(57) N-((2,3-Dihydrobenzofuran) -2-yl)-azolylharnstoffe der Formel

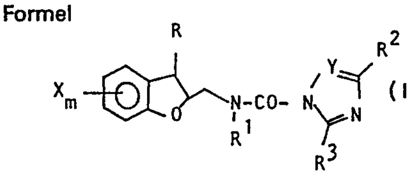

(I),

in der R Wasserstoff oder Alkyl, X Wasserstoff, Halogen, Nitro, Cyan, Trifluormethyl, Alkyl, Alkoxy, Alkylthio, Phenyl oder Phenoxy bedeutet,
m eine ganze Zahl von 1 bis 4 bedeutet,
Y CH oder N bedeutet,
$R^1$ Alkyl, Alkenyl, Alkoxyalkyl, Alkylthioalkyl, Cycloalkyl oder Cycloalkylalkyl oder einen gegebenenfalls substituierten Phenyl- oder Benzylrest bedeutet, und
$R^2$ und $R^3$ Wasserstoff oder Alkyl bedeuten und diese enthaltende Fungizide.

EP 0 156 255 A2

Croydon Printing Company Ltd

N-((2,3-Dihydrobenzofuran)-2-yl)-azolylharnstoffe und diese enthaltende Fungizide

Die vorliegende Erfindung betrifft neue Dihydrobenzofuran-2-yl-azolylharnstoffe, Verfahren zu ihrer Herstellung und fungizide Mittel, die diese Verbindungen als Wirkstoff enthalten.

Es ist bekannt, N-Trichlormethylthio-tetrahydrophthalimid als Fungizid in der Landwirtschaft sowie im Obst- und Gartenanbau einzusetzen (Chem.Week, June 21, 1972, Seite 46). Es ist ferner bekannt, 1-(2-(2,4-Dichlorphenyl)-2--(2-propenyloxy)-ethyl)-1H-imidazol als Fungizid zu verwenden (GB-P 1 318 590). Seine Wirkung ist jedoch, insbesondere bei niedrigen Aufwandmengen, ungenügend. Es besteht daher die Aufgabe, wirksamere fungizide Wirkstoffe zu entwickeln. Das bekannte Mittel ist jedoch nur vor der Infektion verwendbar und seine Wirkung genügt bei niedrigen Aufwandmengen den Anforderungen der Praxis nicht.

Es wurde nun gefunden, daß Dihydrobenzofuran-2-yl-azolylharnstoffe der Formel

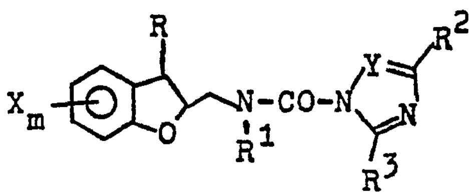 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet, $R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und $R^2$ und $R^3$ gleich oder verschieden sind und Wasserstoff oder Alkyl mit 1 bis 5 C-Atomen bedeuten, sehr gut wirksam gegen Schadpilze sind.

Die neuen Verbindungen der Formel I enthalten chirale Zentren und werden im allgemeinen in Form von Racematen oder als Diastereomerengemische erhalten. Die Diastereomeren lassen sich bei einigen der erfindungsgemäßen Verbindungen beispielsweise durch Säulenchromatographie trennen oder auf-Sws/P

grund von Löslichkeitsunterschieden in reiner Form isolieren. Aus solchen einheitlichen Diastereomeren kann man mit bekannten Methoden die einheitliche Enantiomeren erhalten. Diese Sowie die analogen optisch einheitlichen Verbindungen werden von der vorliegenden Erfindung ebenso wie die Racemate und Diastereomengemische umfaßt. Für die Anwendung der neuen Verbindungen als Fungizide sind sowohl die einheitlichen Diastereomeren bzw. Enantiomeren wie auch deren bei der Synthese anfallenden Gemische geeignet; bevorzugt werden die letzteren verwendet.

X bedeutet vorzugsweise Wasserstoff, Fluor, Chlor, Brom, Cyan, Nitro, Trifluormethyl, Methyl, Ethyl, n-Propyl, Isopropyl, n-Butyl, Isobutyl, sek.-Butyl, tert.-Butyl, Methoxy, Ethoxy, Methylthio, Phenyl oder Phenoxy.

Als Bedeutung für $R^1$ in Formel I kommen beispielsweise Reste wie Methyl, Ethyl, n-Propyl, n-Butyl, Isobutyl, sek.-Butyl, n-Pentyl, 3-Methyl-1--butyl, 2-Methyl-1-butyl, 3-Pentyl, n-Hexyl, 3,3-Dimethyl-1-butyl, 2,2,3--Trimethyl-1-propyl, n-Heptyl, n-Octyl, 2,4,4-Trimethylpentyl, 2-Ethylhexyl, n-Nonyl, 3,5,5-Trimethylhexyl, 2-Isopropyl-5-methylhexyl, 2-Isopropyl-5-methylhexyl, n-Decyl, 3,7-Dimethyloctyl, Dodecyl, Cyclopropylmethyl, Cyclopentyl, Cyclohexyl, Cyclohexylmethyl, 4-Methylcyclohexyl, 4-tert.-Butylcyclohexyl, 4-Methoxycyclohexyl, 2-Methoxyethyl, 2-Ethoxyethyl, 2-Propoxyethyl, 2-Butoxyethyl, 2-Hexyloxyethyl, 3-Methoxypropyl, 3--Ethoxypropyl, 3-Propoxypropyl, 3-Hexyloxypropyl, 2-Methylthioethyl, 2-Ethylthioethyl, 2-Propylthio-ethyl, 2-Butylthio-ethyl, 3-Methylthiopropyl, 3-Ethylthiopropyl, 3-Propylthiopropyl, Allyl, 2-Methylallyl, 2--Buten-1-yl, 2-Penten-1-yl, Phenyl, 4-Fluorphenyl, 4-Chlorphenyl, 2,4--Dichlorphenyl, 3,4-Dichlorphenyl, 4-Bromphenyl, 4-Methylphenyl, 4-tert.--Butylphenyl, 4-Methoxyphenyl, 4-Ethoxyphenyl, 4-Trifluormethylphenyl, 4-Cyanphenyl, Benzyl, 4-Fluorbenzyl, 4-Chlorbenzyl, 3,4-Dichlorbenzyl, 2,4-Dichlorbenzyl, 4-Brombenzyl, 4-Methylbenzyl, 4-tert.-Butylbenzyl, 4-Methoxybenzyl, 3- und 4-Trifluormethylbenzyl und 4-Ethoxybenzyl in Frage

R, $R^2$ und $R^3$ bedeuten vorzugsweise Wasserstoff, Methyl, Ethyl, n-Propyl oder Isopropyl.

Y bedeutet Stickstoff oder CH und m eine ganze Zahl von 1, 2, 3 oder 4.

Die Verbindungen der Formel I lassen sich herstellen, indem man ein Carbamoylchlorid der Formel II

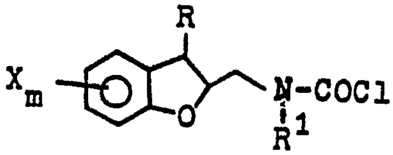 (II),

in der R, $R^1$ und $X_m$ die oben angegebenen Bedeutungen haben,

a) mit Azolen der Formel III

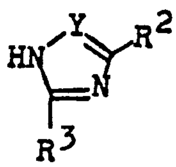 (III),

in der $R^2$, $R^3$ und Y die oben angegebenen Bedeutungen haben, oder

b) mit deren Metall-Derivaten der Formel IV

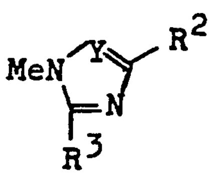 (IV),

in der $R^2$, $R^3$ und Y die oben angegebenen Bedeutungen haben und Me Lithium, Natrium, Kalium oder 1/2-Calcium bedeutet, oder

c) mit deren Silyl-Derivaten der Formel V

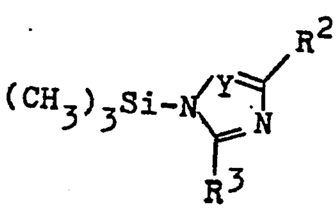 (V),

in der $R^2$, $R^3$ und Y die oben angegebenen Bedeutungen haben, umsetzt.

Die Reaktion a) erfolgt gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120 °C.

Als bevorzugte, gegenüber den Reaktionsteilnehmern inerte Lösungs- oder Verdünnungsmittel können beispielsweise aliphatische oder aromatische, gegebenenfalls halogenierte Kohlenwasserstoffe, wie n-Pentan, Cyclohexan, Methylenchlorid, 1,1,1-Trichlorethan, Benzol, Toluol, Xylol, Chlorbenzol, aliphatische Ketone, wie Aceton, Methylethylketon, Diethylketon, Ether, wie Diethylether, Methyl-tert.-butylether, Dimethoxyethan, Tetrahydrofuran oder Dioxan, Ester, wie Essigsäureethylester, Nitrile wie Acetonitril, Amide, wie Dimethylformamid, Dimethylacetamid und N-Methylpyrrolidon oder Gemische dieser Lösungsmittel verwendet werden.

Geeignete Basen, die gegebenenfalls auch als säurebindende Mittel bei der Reaktion verwendet werden können, sind beispielsweise Alkalihydroxide wie Lithium-, Natrium-, Kalium- oder Calciumhydroxid, Alkalicarbonate wie Natrium- oder Kaliumcarbonat oder Natrium- und Kaliumhydrogencarbonat, Amine wie Triethylamin, Tripropylamin, N-Methylpyrrolidin, N-Methylpiperidin, N,N'-Tetramethylethylendiamin, N,N-Dimethylanilin, N,N--Diethylanilin, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammonium-chlorid, -bromid oder -iodid, Benzyltriethylammonium-chlorid oder -bromid oder Kronenether wie 12-Krone-4, 15-Krone-5, 18-Krone-6 oder Dibenzo-18-krone-6 in Frage.

Die Reaktionen b) und c) erfolgen gegebenenfalls in Gegenwart eines Lösungs- oder Verdünnungsmittels bei einer Temperatur im Bereich zwischen 0 und 140 °C, vorzugsweise zwischen 0 und 100 °C. Hierfür eignen sich dieselben Lösungsmittel wie für die Verfahrensvariante a).

Die Verbindungen der Formel I lassen sich ferner herstellen, indem man ein sekundäres Amin der Formel VI

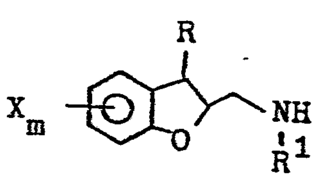 (VI),

in der R, $R^1$ und $X_m$ die oben angegebenen Bedeutungen haben, mit einem Carbonyl-bisazol der Formel VII

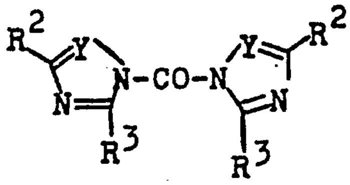
(VII),

in der $R^2$, $R^3$ und Y die oben angegebenen Bedeutungen haben, gegebenenfalls in Gegenwart eines Lösungs- oder Verdünnungsmittels, gegebenenfalls unter Zusatz eines Reaktionsbeschleunigers, umsetzt.

Hierfür eignen sich beispielsweise folgende Lösungs- oder Verdünnungsmittel: Diethylether, 1,2-Dimethoxyethan, Dipropylether, Dibutylether, Methyl-tert.-butylether, Tetrahydrofuran, Dimethoxyethan, Anisol, n-Pentan, n-Hexan, n-Heptan, n-Octan, Isooctan, Cyclohexan, Toluol, Chlorbenzol, Xylole, Acetonitril, Essigester, Dimethylformamid, N-Methylpyrrolidon, Aceton oder Methylethylketon.

Geeignete Reaktionsbeschleuniger sind beispielsweise 4-Dimethylaminopyridin oder 4-Pyrrolidinopyridin.

Die Ausgangsstoffe der Formel II können leicht nach bekannten Verfahren hergestellt werden, beispielsweise durch Umsetzung von Aminen der Formel VI mit Phosgen (Houben-Weyl-Müller, Methoden der organischen Chemie, Band 8, Seite 115 bis 118, Georg Thieme Verlag, Stuttgart, 1952).

Die sekundären Amine der Formel VI werden schließlich durch Umsetzung von bekannten Aminen der Formel $R^1NH_2$, in der $R^1$ die oben angegebene Bedeutung hat, mit 2,3-Dihydrobenzofuran-2-methylhalogeniden (s. Toyashima und Mitarbeiter, Yakugaku Zasshi, 88, 503 (1968)) der Formel VIII

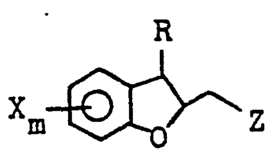
(VIII),

in der R und $X_m$ die oben angegebenen Bedeutungen haben und Z Chlor oder Brom bedeutet, gegebenenfalls in Gegenwart einer starken anorganischen

oder organischen Base und gegebenenfalls in Gegenwart eines Lösungs- oder Verdünnungsmittels hergestellt.

Die folgenden Beispiele erläutern die Herstellung der Verbindungen der Formel I:

Beispiel 1

a) 33 g (0,118 Mol) 5,7-Dichlor-2,3-dihydrobenzofuran-2-ylmethylbromid und 200 ml n-Butylamin werden bei 60 °C 12 Stunden gerührt. Das Gemisch wird unter vermindertem Druck eingeengt und der Rückstand mit 100 ml einer 20 %igen wäßrigen Natriumhydroxidlösung versetzt und anschließend mit 300 ml Methylenchlorid ausgeschüttelt. Die organische Phase wird dreimal mit je 70 ml Wasser gewaschen, getrocknet und eingeengt. Der Rückstand wird in 300 ml trockenem Diethylether gelöst und bei 0 bis +3 °C mit trockenem Chlorwasserstoff begast. Der farblose, kristalline Niederschlag wird abgesaugt, mit trockenem Diethylether gewaschen und getrocknet. Man erhält 27 g N-(5,7-Dichlor-2,3-dihydrobenzofuran-2-ylmethyl)-N-butylaminchlorhydrat vom Schmp. 202 - 205 °C.

b) Eine Suspension von 26 g (0,0838 Mol) N-(5,7-Dichlor-2,3-dihydrobenzofuran-2-ylmethyl)-N-butylaminchlorhydrat in 250 ml trockenem Essigester wird bei 50 °C unter gutem Rühren mit Phosgen begast. Nach 4 Stunden löst sich die Suspension auf. Das Gemisch wird im Vakuum, schließlich bei 50 °C und 0,5 mbar eingeengt. Man erhält 27 g N-(5,7-Dichlor-2,3-dihydrobenzofuran-2-ylmethyl)-N-butylcarbamoylchlorid als farbloses Öl, das ohne zusätzliche Reinigung weiter umgesetzt wird.

c) Zu einer Lösung von 6,8 g (0,1 Mol) Imidazol in 150 ml trockenem Tetrahydrofuran wurden bei 20 °C 13,5 g (0,04 Mol) N-(5,7-Dichlor-2,3-dihydrobenzofuran-2-ylmethyl)-N-butylcarbamoylchlorid zugetropft. Nach 6stündigem Rühren bei 70 °C wurde das Gemisch auf 20 °C abgekühlt und der gebildete Niederschlag abgesaugt. Das Filtrat wurde unter vermindertem Druck eingeengt, der Rückstand in 200 ml Methylenchlorid gelöst, dreimal mit je 80 ml Wasser gewaschen, getrocknet und eingeengt. Das verbleibende Harz wurde mit 30 ml Diethylether bei 0 °C 3 Stunden stehengelassen und die gebildete Kristalle abgesaugt. Man erhielt 12,8 g 1-(N-(5,7-Dichlor-2,3-dihydrobenzofuran-2-ylmethyl)-N-butylcarbamoyl)-imidazol vom Schmp. 98 bis 100°C (Verbindung Nr. 1).

Beispiel 2

Zu einer Suspension von 5,5 g (0,06 Mol) Natrium-1,2,4-triazolid in 120 ml trockenem Tetrahydrofuran wurden bei 20 °C 13,5 g (0,04 Mol) N-(5,7-Dichlor-2,3-dihydrobenzofuran-2-ylmethyl)-N-butylcarbamoylchlorid zugetropft. Nach 6stündigem Rühren bei 65 °C wurde das Gemisch auf 20 °C abgekühlt und der Niederschlag abgesaugt. Das Filtrat wurde eingeengt und der Rückstand mit 30 ml Diethylether aufgeschlämmt und abgesaugt. Man erhielt 13,1 g 1-(N-(5,7-Dichlor-2,3-dihydrobenzofuran-2-ylmethyl)-N-butylcarbamoyl)-1,2,4-triazol als farblose Kristalle vom Schmp. 122 bis 124°C (Verbindung Nr. 2).

In entsprechender Weise können die in der folgenden Tabelle aufgeführten Verbindungen hergestellt werden:

| Beispiel Nr. | $X_m$ | R | $R^1$ | Y | $R^2$ | $R^3$ | Fp [°C] IR (Film) $cm^{-1}$ |
|---|---|---|---|---|---|---|---|
| 3 | H | H | $n-C_3H_7$ | CH | H | H | 1693, 1480, 1462, 1418, 1296, 1232, 1100, 1070, 1002, 753, 658 |
| 4 | H | H | $n-C_3H_7$ | N | H | H | 72 - 74 |
| 5 | H | H | $-(CH_2)_2-O-C_2H_5$ | CH | H | H | |
| 6 | H | $CH_3$ | $-(CH_2)_2-O-C_3H_7-n$ | CH | H | H | |
| 7 | H | $CH_3$ | $-(CH_2)_2-O-C_6H_{13}-n$ | CH | H | H | |
| 8 | H | $C_2H_5$ | $-(CH_2)_3-O-CH_3$ | CH | H | H | |
| 9 | H | $C_2H_5$ | $-(CH_2)_3-OC_2H_5$ | CH | H | H | |
| 10 | H | H | $-(CH_2)_3-OC_2H_5$ | CH | H | H | |
| 11 | H | H | $-(CH_2)_3-OC_3H_7$ | CH | H | H | |
| 12 | H | H | Cyclohexyl | CH | H | H | 88 - 90 |
| 13 | H | H | Cyclohexyl | N | H | H | 2932, 1698, 1481, 1462, 1420, 1374, 1227, 1183, 1016, 991, 750, 660 |
| 14 | H | H | $n-C_3H_7$ | $C-CH_3$ | $CH_3$ | $CH_3$ | 2963, 1691, 1646, 1481, 1463, 1362, 1230, 1100, 1017, 872, 751 |
| 15 | H | H | $n-C_6H_{13}$ | CH | | H | 2955, 1694, 1481, 1418, 1295, 1282, 1230, 1000, 752, 658 |
| 16 | H | H | $n-C_6H_{13}$ | N | N | H | 2929, 1701, 1480, 1463, 1424, 1380, 1277, 1230, 992, 872, 750 |

| Beispiel Nr. | $X_m$ | R | $R^1$ | Y | $R^2$ | $R^3$ | Fp [°C] IR (Film) $cm^{-1}$ |
|---|---|---|---|---|---|---|---|
| 17 | H | H | $-CH_2-CH=CH-CH_3$ | CH | H | H | |
| 18 | H | H | $-CH_2-CH=CH-CH_3$ | N | H | H | |
| 19 | H | H | $-CH_2-CH=CH-C_3H_7$ | CH | H | H | |
| 20 | H | H | $C_6H_5$ | CH | H | H | |
| 21 | H | H | $C_6H_5$ | N | H | H | |
| 22 | H | H | $4-CH_3-C_6H_4-$ | CH | H | H | |
| 23 | 5-F | H | $n-C_6H_{13}$ | CH | H | H | |
| 24 | 5-F | H | $n-C_6H_{13}$ | N | H | H | |
| 25 | 5-Cl | H | $n-C_6H_{13}$ | CH | H | H | |
| 26 | 5-Cl | H | $n-C_6H_{13}$ | N | H | H | |
| 27 | $5,7-Cl_2$ | H | $n-C_3H_7$ | CH | H | H | 108 - 110 |
| 28 | $5,7-Cl_2$ | H | $n-C_3H_7$ | N | H | H | 99 - 101 |
| 29 | $5,7-Cl_2$ | H | $iso-C_4H_9$ | CH | H | H | 131 - 132 |
| 30 | $5,7-Cl_2$ | H | $iso-C_4H_9$ | N | H | H | 73 - 75 |
| 31 | $5,7-Cl_2$ | H | $sek.-C_4H_9$ | CH | H | H | 1698, 1462, 1378, 1300, 1275, 1197, 991, 762, 670 |
| 32 | $5,7-Cl_2$ | H | $sek.-C_4H_9$ | N | H | H | 1676, 1460, 1413, 1375, 1334, 1300, 1161, 996, 851, 758 |
| 33 | $5,7-Cl_2$ | H | $n-C_6H_{13}$ | CH | H | H | 80-82 |
| 34 | $5,7-Cl_2$ | H | $n-C_6H_{13}$ | N | H | H | 79-82 |
| 35 | $5,7-Cl_2$ | H | $-(CH_2)_3-O-C_4H_9-iso$ | CH | H | H | 61-63 |
| 36 | $5,7-Cl_2$ | H | $-(CH_2)_3-O-C_4H_9-iso$ | N | H | H | 90-92 |

| Beispiel Nr. | $X_m$ | R | $R^1$ | Y | $R^2$ | $R^3$ | Fp. [°C] IR (Film) $cm^{-1}$ |
|---|---|---|---|---|---|---|---|
| 37 | 5,7-$Cl_2$ | H | $-CH_2-CH(C_2H_5)-C_4H_9$-n | CH | H | H | 121 - 123 |
| 38 | 5,7-$Cl_2$ | H | $-(CH_2)_2-CH(CH_3)-C(CH_3)_3$ | CH | H | H | 84 - 85 |
| 39 | 5,7-$Cl_2$ | H | $-(CH_2)_2-CH(CH_3)-C(CH_3)_3$ | N | H | H | $n_D^{26}$ = 1,5340 |
| 40 | 5,7-$Cl_2$ | H | $-CH_2-CH(C_2H_5)-C_4H_9$-n | N | H | H | 50 - 53 |
| 41 | 5,7-$Cl_2$ | H | $-CH_2$-Cyclopropyl | CH | H | H | 114 - 116 |
| 42 | 5,7-$Cl_2$ | H | $-CH_2$-Cyclopropyl | N | H | H | 86 - 88 |
| 43 | 5,7-$Cl_2$ | H | $-(CH_2)_2-O-CH_3$ | CH | H | H | 84 - 86 |
| 44 | 5,7-$Cl_2$ | H | $-(CH_2)_2-O-CH_3$ | N | H | H | 129 - 132 |
| 45 | 5,7-$Cl_2$ | H | $-(CH_2)_2-S-CH_3$ | CH | H | H | 67 - 120 |
| 46 | 5,7-$Cl_2$ | H | $-(CH_2)_2-S-CH_3$ | N | H | H | 118 - 120 |
| 47 | 5,7-$Cl_2$ | H | $-CH_2-CH=CH_2$ | CH | H | H | 113 - 115 |
| 48 | 5,7-$Cl_2$ | H | $-CH_2-CH=CH_2$ | N | H | H | 83 - 85 |
| 49 | 5-Br, 7-Cl | H | n-$C_4H_9$ | N | H | H | |
| 50 | 5-Br, 7-Cl | H | n-$C_4H_9$ | CH | H | H | 98 -100 |
| 51 | 4,6-$Cl_2$ | H | n-$C_6H_{13}$ | CH | H | H | |
| 52 | 4,6-$Cl_2$ | H | n-$C_6H_{13}$ | N | H | H | |
| 53 | 5-$CH_3$ | H | n-$C_6H_{13}$ | CH | H | H | |
| 54 | 5-$CH_3$ | H | n-$C_6H_{13}$ | N | H | H | |
| 55 | 5-tert.-$C_4H_9$ | H | n-$C_6H_{13}$ | CH | H | H | |
| 56 | 5-tert.-$C_4H_9$ | H | n-$C_6H_{13}$ | N | H | H | |
| 57 | 5-$CH_3O$- | H | n-$C_4H_9$ | CH | H | H | 1696, 1494, 1446, 1252, 1233, 1197, 995, 846, 823, 768 |

| Beispiel Nr. | $X_m$ | R | $R^1$ | Y | $R^2$ | $R^3$ | Fp [°C] IR (Film) $cm^{-1}$ |
|---|---|---|---|---|---|---|---|
| 58 | $5-CH_3O-$ | H | $n-C_4H_9$ | N | H | H | 1700, 1489, 1429, 1380, 1277, 1219, 1203, 1183, 1047, 993, 810, 744 |
| 59 | $5-C_2H_5-O-$ | H | $n-C_6H_{13}$ | CH | H | H | |
| 60 | $5\ C_2H_5-O-$ | H | $n-C_6H_{13}$ | N | H | H | |
| 61 | $5-CH_3O$ | H | $n-C_6H_{13}$ | CH | H | H | 1703, 1496, 1457, 1417, 1296, 1251, 1234, 1189, 1000, 850, 822 |
| 62 | $5-CH_3O-$ | H | $n-C_6H_{13}$ | N | H | H | 1700, 1488, 1468, 1430, 1380, 1276, 1204, 1134, 1030, 992, 740 |
| 63 | H | H | $-CH(CH_3)C_5H_{11}-n$ | CH | H | H | 2929, 1697 1481, 1461, 1416, 1377, 1229, 991, 750 |
| 64 | H | H | $-CH(CH_3)C_5H_{11}-n$ | N | H | H | 2955, 1692, 1480, 1408, 1370, 1234, 1099, 752 |
| 65 | H | H | Cyclooctyl | CH | H | H | 2922, 1693, 1480, 1448, 1376, 1232, 1068, 1000, 751 |
| 66 | H | H | Cyclooctyl | N | H | H | 2922, 1700, 1480, 1462, 1377, 1228, 993, 749, 670 |

Die neuen Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden. Ferner können sie auch im Materialschutz, u. a. zur Bekämpfung holzzerstörender Pilze, wie Coniophora puteana und Polystictus versicolor, verwendet werden. Darüber hinaus können auch holzverbläuende Pilze wie Pullularia pullulans und Schimmelpilze mit den neuen Wirkstoffen bekämpft werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie Gemüse - wie Gurken, Bohnen und Kürbisgewächse -.

Die neuen Verbindungen sind insbesondere geeignet zur Bekämpfung folgender Pflanzenkrankheiten:

Pseudocercosporella herpotrichoides in Getreide, Erysiphe graminis (echter Mehltau) in Getreide, Erysiphe cichoriacearum an Kürbisgewächsen, Podosphaera leucotricha an Äpfeln, Uncinula necator an Reben,
Puccinia-Arten an Getreide,
Rhizoctonia solani an Baumwolle,
Ustilage-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Rhynchosporium secalis und
Pyrenophora teres in Getreide.

Die Wirkstoffe können gleichzeitig das Wachstum von zwei oder mehr der genannten Pilze unterdrücken und besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirksungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den neuen Verbindungen kombiniert werden können, sind beispielsweise:

Schwefel,
Dithiocarbamate und deren Derivate, wie
Ferridimethyldithiocarbamat, Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester

N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxyethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol.

Die neuen Wirkstoffe werden beispielsweise in Form von direkt versprühbaren Lösungen, Suspensionen, Emulsionen, auch in Form von hochprozentigen wäßrigen, öligen oder sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw. stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze

der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind:

I. Man vermischt 90 Gewichtsteile der Verbindung 31 mit 100 Gewichtsteilen N-Methylpyrrolidon und erhält eine Lösung, die zur Anwendung in Form kleinster Tropfen geeignet ist.

II. 20 Gewichtsteile der Verbindung 12 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Druch Ausgießen und feines Verteilen der Lösung in Wasser erhält man eine wäßrige Dispersion.

III. 20 Gewichtsteile der Verbindung 15 werden in einer Mischung gelöst, die aus 30 Gewichtsteilen Cyclohexanon, 30 Gewichtsteilen Isobutanol, 20 Gewichtsteilen des Anlagerungsproduktes von 40 Mol

Ethylenoxid an 1 Mol Ricinusöl besteht. Durch Eingießen und Verrühren der Lösung in Wasser erhält man eine wäßrige Dispersion.

IV. 20 Gewichtsteile der Verbindung 16 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der Verbindung 28 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der Verbindung 30 werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der Verbindung 31 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 43 werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der Verbindung 44 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel (A) ist der bekannte, insbesondere zur Bekämpfung von Botrytis geeignete Wirkstoff N-Trichlormethylthio-tetrahydrophthalimid (Chem. Week, June 21, 1972, Seite 46). Vergleichsmittel B ist der Wirkstoff 1-(2-(2,4-Dichlorphenyl)-2-(2-propenyloxy)-ethyl)-1H-imidazol (GB-13 18 590).

Versuch 1

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05 %ige Wirkstoffbrühe der Wirkstoff 31 eine bessere fungizide Wirkung zeigt (beispielsweise 90 %) als der bekannte Wirkstoff A (beispielsweise 70 %).

Versuch 2

Wirksamkeit gegen Pyricularia oryzae

Blätter von in Töpfen gewachsenen Reiskeimlingen der Sorte "Bahia" werden mit wäßrigen Emulsionen, die 80 % Wirkstoff und 20 % Emulgiermittel in der Trockensubstanz enthalten, besprüht und 24 Stunden später mit einer wäßrigen Sporensuspension von Pyricularia oryzae inoculiert. Die Versuchspflanzen werden in Klimakammern bei 22 bis 24°C und 95 bis 99 % relativer Luftfeuchtigkeit aufgestellt. Nach 7 Tagen wird das Ausmaß der Pflanzenkrankheit ermittelt.

Das Ergebnis des Versuches zeigt, daß beispielsweise bei der Anwendung als 0,05 %ige Wirkstoffbrühe die Wirkstoffe 12, 15, 16, 28, 30, 31, 43, 44 und 48 eine gute fungizide Wirkung zeigten (beispielsweise 90 %).

Versuch 3

Wirksamkeit gegen Weizenbraunrost

Blätter von in Töpfen gewachsenen Weizensämlingen der Sorte "Jubilar" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten,

tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise bei der Anwendung als 0,05 %ige Wirkstoffbrühe die Wirkstoffe 27, 29 und 31 eine bessere fungizide Wirkung zeigten (beispielsweise 97 %) als der bekannte Wirkstoff B (beispielsweise 70 %).

Versuch 4

Wirksamkeit gegen Halmbruchkrankheit an Weizen

Blätter von in Töpfen gewachsenen Weizenkeimlingen der Sorte "Jubilar" werden mit wäßrigen Emulsionen, die 80 % Wirkstoff und 20 % Emulgator in der Trockensubstanz enthalten, bis zur Tropfnässe besprüht und 24 Stunden später mit einer wäßrigen Sporensuspension des Erregers der Halmbruchkrankheit - Pseudocercosporella herpotrichoides - inoculiert. Anschließend werden die Versuchspflanzen in Klimakammern bei 95 bis 99 % relativer Luftfeuchtigkeit und 8 bis 10°C aufgestellt. Nach 3 bis 4 Wochen weist der überwiegende Teil der unbehandelten Kontrollpflanzen deutliche Krankheitssymptome auf, so daß die Auswertung des Versuches vorgenommen werden kann.

Das Ergebnis des Versuches zeigt, daß beispielsweise bei der Anwendung als 0,01 %ige Wirkstoffbrühe der Wirkstoff 15 eine gute fungizide Wirkung zeigt (beispielsweise 90 %).

Patentansprüche

1. N-((2,3-Dihydrobenzofuran)-2-yl)-azolylharnstoff der Formel

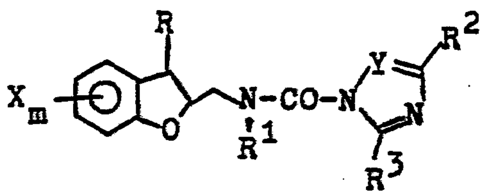

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

2. Fungizides Mittel, enthaltend N-((2,3-Dihydrobenzofuran)-2-yl)-azolylharnstoff der Formel

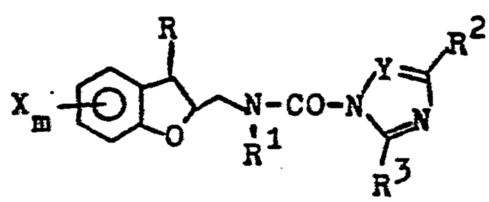

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen

jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

3. Fungizides Mittel, enthaltend einen inerten Zusatzstoff und N-((2,3-Dihydrobenzofuran)-2-yl)-azolylharnstoff der Formel

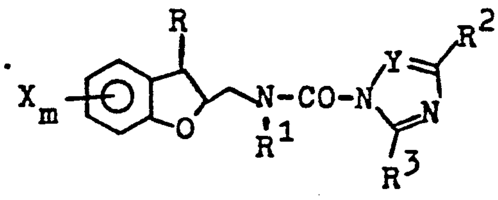 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

4. Verfahren zur Herstellung eines fungiziden Mittels, dadurch gekennzeichnt, daß man einen inerten Zusatzstoff vermischt mit N-((2,3-Dihydrobenzofuran)-2-yl)-azolylharnstoff der Formel

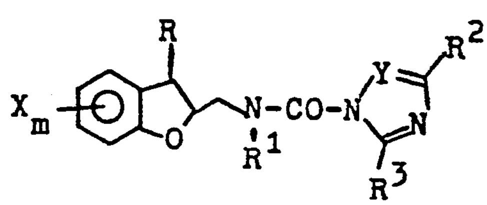 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

5. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge N-((2,3-Dihydrobenzofuran)-2-yl)-azolylharnstoff der Formel

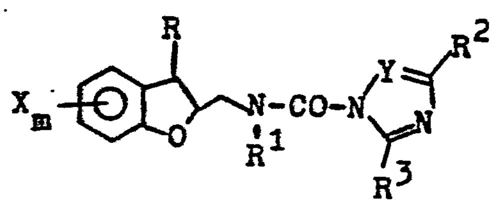

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

6. Verfahren zur Herstellung von N-((2,3-Dihydrobenzofuran)-2-yl)--azolylharnstoff der Formel

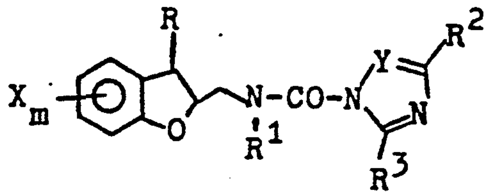 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten, <u>dadurch gekennzeichnet</u>, daß man eine Verbindung der Formel

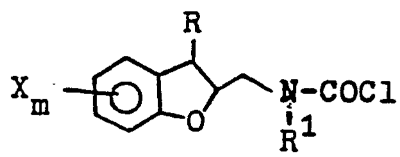 (II),

in der R, $R^1$ und $X_m$ die im Anspruch 1 angegebenen Bedeutungen haben,

a) mit Azolen der Formel

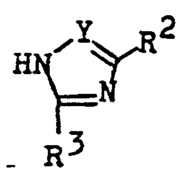 (III),

in der $R^2$, $R^3$ und Y die in Anspruch 1 angegebenen Bedeutungen haben, oder

b) mit deren Metall-Derivaten der Formel

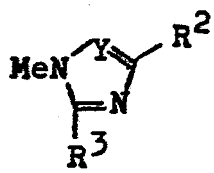 (IV),

in der $R^2$, $R^3$ und Y die im Anspruch 1 angegebenen Bedeutungen haben und Me Lithium, Natrium, Kalium oder 1/2-Calcium bedeutet, oder

c) mit deren Silyl-Derivaten der Formel

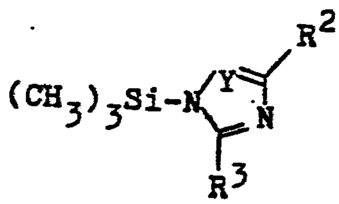 (V),

in der $R^2$, $R^3$ und Y die im Anspruch 1 angegebenen Bedeutungen haben, umsetzt.

7. Verfahren zur Herstellung von N-((2,3-Dihydrobenzofuran)-2-yl)-azolylharnstoff der Formel

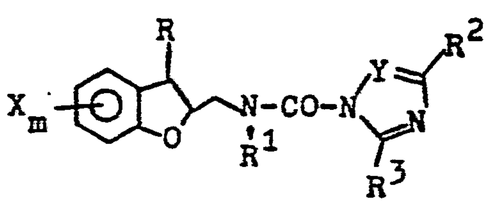 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder

Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten, dadurch gekennzeichnet, daß man eine Verbindung der Formel

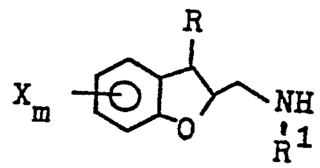 (VI),

in der R, $R^1$ und $X_m$ die im Anspruch 1 angegebenen Bedeutungen haben, mit einem Carbonyl-bisazol der Formel

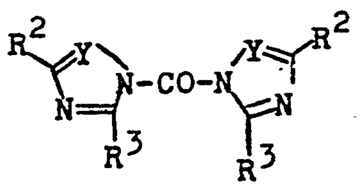 (VII),

in der $R^2$, $R^3$ und Y die im Anspruch 1 angegebenen Bedeutungen haben, umsetzt.

8. Dihydrobenzofuran-2-yl-azolylharnstoffe der Formel I gemäß Anspruch 1, dadurch gekennzeichnet, daß X Wasserstoff, Fluor, Chlor, Brom, Cyan, Nitro, Trifluormethyl, Methyl, Ethyl, n-Propyl, Isopropyl, n-Butyl, Isobutyl, sek.-Butyl, tert.-Butyl, Methoxy, Ethoxy, Methylthio, Phenyl, oder Phenoxy bedeutet, R, $R^2$ und $R^3$ Wasserstoff, Methyl, Ethyl, n-Propyl oder Isopropyl bedeuten, Y CH oder N ist,

$R^1$ Methyl, Ethyl, n-Propyl, n-Butyl, Isobutyl, sek.-Butyl, n-Pentyl, 3-Methyl-1-butyl, 2-Methyl-1-butyl, 3-Pentyl, n-Hexyl, 3,3-Dimethyl-1-butyl, 2,2,3-Trimethylpropyl-1, n-Heptyl, n-Octyl, 2,4,4-Trimethylpentyl, 2-Ethylhexyl, n-Nonyl, 3,5,5-Trimethylhexyl, 2-Isopropyl-5-methylhexyl, n-Decyl, 3,7-Dimethyloctyl, Cyclopropylmethyl, Cyclopentyl, Cyclohexyl, Cyclohexylmethyl, 4-Methylcyclohexyl, 2-Methoxyethyl, 2-Ethoxyethyl, 2-Propoxyethyl, 2-Butoxyethyl, 3-Methoxypropyl, 3-Ethoxypropyl, 3-Propoxypropyl, 3-Butoxypropyl, 2-Methylthio-ethyl, 2-Ethylthioethyl, 3-Methylthiopropyl, Allyl, 2-Buten-1-yl, 2-Penten-1-yl, Phenyl, p-Chlorphenyl, p-Methoxyphenyl, p-Methylphenyl, Benzyl, p-Chlorbenzyl, 2,4-Dichlorbenzyl, p-Methylbenzyl oder Phenylethyl bedeutet und m eine ganze Zahl von 1 bis 4 ist.

0156255

Patentansprüche

1. Fungizides Mittel, enthaltend N-((2,3-Dihydrobenzofuran)-2-yl)-azolylharnstoff der Formel

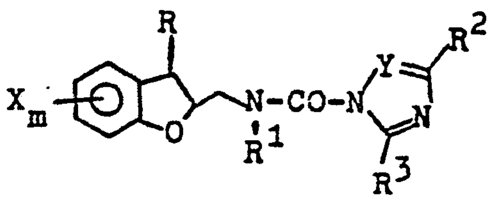

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

2. Fungizides Mittel, enthaltend einen inerten Zusatzstoff und N-((2,3-Dihydrobenzofuran)-2-yl)-azolylharnstoff der Formel

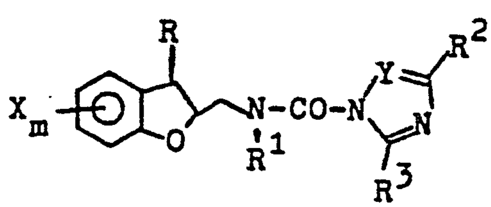

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,

$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

3. Verfahren zur Herstellung eines fungiziden Mittels, dadurch gekennzeichnt, daß man einen inerten Zusatzstoff vermischt mit N-((2,3-Dihydrobenzofuran)-2-yl)-azolylharnstoff der Formel

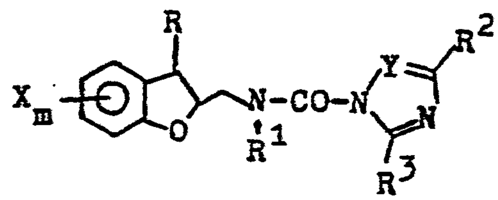 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

4. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge N-((2,3-Dihydrobenzofuran)-2-yl)-azolylharnstoff der Formel

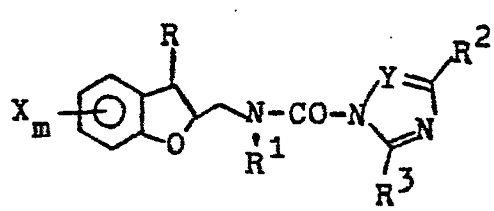 (I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten.

5. Verfahren zur Herstellung von N-((2,3-Dihydrobenzofuran)-2-yl)-azolylharnstoff der Formel

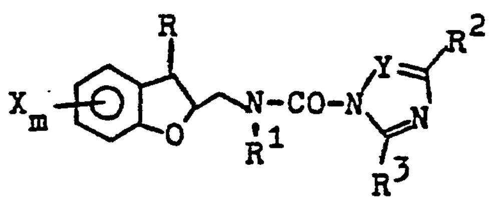
(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten, <u>dadurch gekennzeichnet</u>, daß man eine Verbindung der Formel

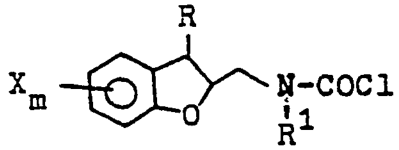
(II),

in der R, $R^1$ und $X_m$ die im Anspruch 1 angegebenen Bedeutungen haben,

a) mit Azolen der Formel

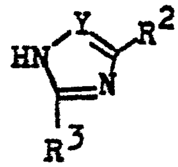 (III),

in der $R^2$, $R^3$ und Y die in Anspruch 1 angegebenen Bedeutungen haben, oder

b) mit deren Metall-Derivaten der Formel

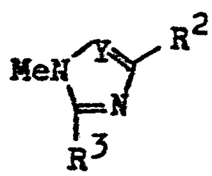 (IV),

in der $R^2$, $R^3$ und Y die im Anspruch 1 angegebenen Bedeutungen haben und Me Lithium, Natrium, Kalium oder 1/2-Calcium bedeutet, oder

c) mit deren Silyl-Derivaten der Formel

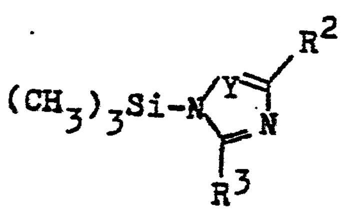 (V),

in der $R^2$, $R^3$ und Y die im Anspruch 1 angegebenen Bedeutungen haben, umsetzt.

6. Verfahren zur Herstellung von N-((2,3-Dihydrobenzofuran)-2-yl)-azolylharnstoff der Formel

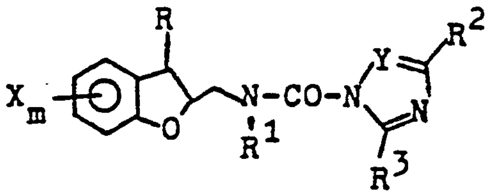

(I),

in der R Wasserstoff oder Alkyl mit 1 bis 4 C-Atomen bedeutet, X Wasserstoff, Halogen, Nitro, Cyan, Trifluormethyl oder Alkyl, Alkoxy und Alkylthio jeweils mit 1 bis 4 C-Atomen oder Phenyl oder Phenoxy bedeutet, m eine ganze Zahl von 1 bis 4 bedeutet, wobei die einzelnen Gruppen X gleich oder verschieden sind, wenn m größer als 1 ist, Y CH oder N bedeutet,
$R^1$ Alkyl, Alkenyl, Alkoxyalkyl, Alkylthioalkyl, Cycloalkyl oder Cycloalkylalkyl jeweils mit bis zu 12 Kohlenstoffatomen oder einen jeweils gegebenenfalls durch Halogen, $C_1$-$C_4$-Alkyl, $C_1$-$C_4$-Alkoxy, Trifluormethyl, Nitro oder Cyan substituierten Phenyl- oder Benzylrest bedeutet, und
$R^2$ und $R^3$ gleich oder verschieden sind und Wasserstoff oder Alkyl mit 1 bis 5 C-Atomen bedeuten, dadurch gekennzeichnet, daß man eine Verbindung der Formel

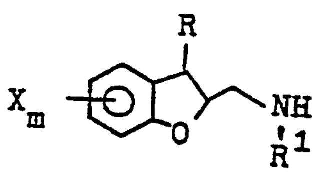

(VI),

in der R, $R^1$ und $X_m$ die im Anspruch 1 angegebenen Bedeutungen haben, mit einem Carbonyl-bisazol der Formel

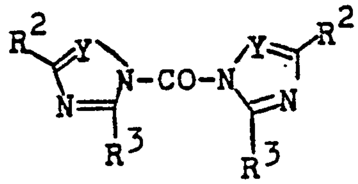

(VII),

in der $R^2$, $R^3$ und Y die im Anspruch 1 angegebenen Bedeutungen haben, umsetzt.